Europäisches Patentamt

European Patent Office

Office européen des brevets

(11) Veröffentlichungsnummer: **0 208 225**
**B1**

(12) # EUROPÄISCHE PATENTSCHRIFT

(45) Veröffentlichungstag der Patentschrift:
28.03.90

(21) Anmeldenummer: 86108898.7

(22) Anmeldetag: 30.06.86

(51) Int. Cl.⁴: **G01T 1/00**, G01T 1/164,
A61B 6/03

(54) **Röntgendetektorsystem.**

(30) Priorität: **12.07.85 DE 3524975**

(43) Veröffentlichungstag der Anmeldung:
**14.01.87 Patentblatt 87/3**

(45) Bekanntmachung des Hinweises auf die Patenterteilung:
**28.03.90 Patentblatt 90/13**

(84) Benannte Vertragsstaaten:
**DE FR**

(56) Entgegenhaltungen:
**DE-A- 2 814 242**
**DE-A- 3 247 204**
**GB-A- 2 013 877**

(73) Patentinhaber: **Siemens Aktiengesellschaft,
Wittelsbacherplatz 2, D-8000 München 2(DE)**

(72) Erfinder: **Klein, Sigismund, Hassfurter Strasse 8,
D-8500 Nürnberg(DE)**
Erfinder: **Rühle, Wolfgang, Dr. Dipl.-Phys.,
Heckenweg 25A, D-8520 Erlangen(DE)**

## Beschreibung

Die Erfindung betrifft ein Röntgendetektorsystem nach dem Oberbegriff des Patentanspruches.

Röntgendetektorsysteme dieser Art werden beispielsweise für Strahlendiagnostikgeräte benötigt, bei denen eine durch das Gerät erzeugte Röntgenstrahlung einen Untersuchungskörper durchdringt und diese anschließend auf die Intensitätsverteilung untersucht werden soll. Ein Röntgendetektorsystem, wie es z.B. in der Computertomographie und Computerradiographie Anwendung findet, besteht aus mehreren 100 bis zu einigen 1000 Einzeldetektorelementen, die linear oder kreisförmig angeordnet und auf die Röntgenröhre ausgerichtet sind. Dabei ist vor diesem Detektorarray noch ein Kollimator angeordnet, bestehend aus auf die Röntgenröhre ausgerichteten Blechen aus stark absorbierendem Material, um die im Meßfeld erzeugte Streustrahlung zu unterdrücken. Durch dieses Detektorsystem sollten möglichst alle Röntgenquanten, die auf geradlinigem Weg durch das Meßfeld ankommen, nachgewiesen werden; möglichst wenige sollten durch den geometrischen Aufbau des Arrays aus einzelnen Detektorelementen und den Kollimator verlorengehen. Dies ist vorteilhaft, da bei fester Röhrenleistung die Zahl der nachgewiesenen Röntgenquanten z.B. die Bildqualität bestimmt. Bei Erhöhung der Röhrenleistung und/oder Verlängerung der Aufnahmezeit, wodurch eine unvollständige Absorption kompensiert werden könnte, nehmen z.B. die Strahlenbelastung des Patienten, die Röhrenbelastung und/oder Bewegungsartefakte zu.

Durch die DE-PS 2 840 965 ist ein Detektorsystem bekannt, bei dem das Detektorfeld in einem Kreisbogen um den Röhrenfokus angeornet ist, wobei die Detektorelemente teilweise in die für sie vorgesehenen Kollimatorschächte eingelassen sind. Die Wände der Kollimatorschächte schirmen die Detektorelemente gegen eine eventuell auftretende seitliche Streustrahlung ab.

Aus der Figur 4 der DE-PS 2 840 965 ist ersichtlich, daß die einen Kollimatorschacht durchsetzende Strahlung nur zu einem Teil auf die wirksame Detektorfläche, den Szintillationskristall, fällt. Ein nicht unerheblicher Teil der Röntgenstrahlung geht an dem Szintillationskristall vorbei und ist damit einem Nachweis nicht mehr zugänglich. Auch die Einkapselungen (Spalte 4, Zeilen 31 bis 34) des Szintillationskristalls bilden gewissermaßen tote Zonen, in der keine Röntgenstrahlung mehr nachgewiesen werden kann.

Der Füllfaktor F ist ein Maß für die tatsächlich auf die Detektorelemente fallenden Röntgenquanten, bezogen auf die insgesamt am Detektor ankommenden Röntgenquanten. Es ist daher wünschenswert, zur Erzielung eines höheren Füllfaktors die seitlichen toten Zonen (seitlichen Detektorbereiche, die eine auftreffende Strahlung nicht registrieren) weitgehend zu vermeiden und die wirksame Detektorfläche durch Vergrößerung der von der Röntgenstrahlung getroffenen strahlungselektrischen Wandler zu erhöhen. Dadurch soll ein Höchstmaß der die Kollimatorschächte parallel durchlaufenden Röntgenquanten nachgewiesen werden.

Durch die DE-OS 3 247 204 ist ein Röntgendetektorsystem bekannt, bei dem der Füllfaktor gegenüber der Anordnung nach der DE-OS 3 840 965 vergrößert ist. Hierzu ist ein Szintillator im unteren, zur Aufnahme des Szintillators erweiterten Bereich eines Kollimatorschachtes angeordnet. Durch diese Anordnung wird erreicht, daß die Fläche des Detektorelements (Szintillator), auf der die den Kollimatorschacht durchsetzende Strahlung auftrifft, der Querschnittsfläche des Kollimatorschachtes entspricht, wodurch der Füllfaktor erhöht ist. Ein lichtelektrischer Wandler ist hinter dem Szintillator und hinter dem Kollimatorschacht angeordnet und steht über ein optisches Koppelmedium mit dem Szintillator in Verbindung. Eine solche Vorrichtung ist in der Herstellung sehr aufwendig, da in jede Kollimatorplatte eine Aussparung als Erweiterung eingearbeitet werden muß. Zudem ist der Detektor von zwei Elementen (Szintillator und lichtelektrischer Wandler) gebildet, die zueinander justiert werden müssen. Hierzu sind Führungen vorgesehen, die den Herstellungsaufwand vergrößern. Da Führungen nicht ganz spielfrei sind, ist es schwierig, den Szintillator und den lichtelektrischen Wandler exakt zueinander zu positionieren, wodurch die Detektorelemente unterschiedliche Charakteristiken aufweisen können. Im optischen Koppelmedium entstehen Lichtverluste, wodurch die Güte des Detektorsystems verringert ist.

Aus der DE-OS 2 814 242 ist ein Schichtgerät zur Herstellung von Transversalschichtbildern gemäß dem Oberbegriff des Patentanspruches bekannt, bei dem die Detektoren, die von einem Halbleiterdetektor und einem vorgeschalteten Szintillationskristall gebildet sein können, hinter dem Kollimator angeordnet sind.

Der Erfindung liegt die Aufgabe zugrunde, bei einem Röntgendetektorsystem der eingangs genannten Art den Füllfaktor bei einfachem Aufbau des Detektorsystems zu verbessern.

Die Aufgabe ist erfindungsgemäß durch die kennzeichnenden Merkmale des Patentanspruches gelöst.

Bei dem erfindungsgemäß ausgestalteten Röntgendetektorsystem bildet ein Szintillator und ein lichtelektrischer Wandler eine Einheit, die in einem gemeinsamen Gehäuse angeordnet sind. Damit kann eine Ankopplung durch ein optisches Koppelmedium entfallen, so daß die dadurch bedingten Lichtverluste nicht auftreten. Zudem sind der Szintillator und der lichtelektrische Wandler exakt zueinander positioniert, es kann auf Justierelemente und die damit notwendige Justierung verzichtet werden; die Eigenschaften eines solchen Elementes sind genau bestimmbar. Die seitlichen Gehäusewandungen liegen im Strahlungsschatten der Kollimatorbleche, so daß der Füllfaktor und die Packungsdichte der Detektorelemente groß ist.

Die Erfindung ist nachfolgend anhand in der Zeichnung dargestellter Ausführungsbeispiele näher erläutert. Es zeigen:

Fig. 1 eine schematische Darstellung eines Röntgendetektorsystems mit linearer Anordnung der Detektorelemente, und

Fig. 2 eine schematische Darstellung eines Röntgendetektorsystems nach der Erfindung mit alternierender Anordnung der Detektorelemente.

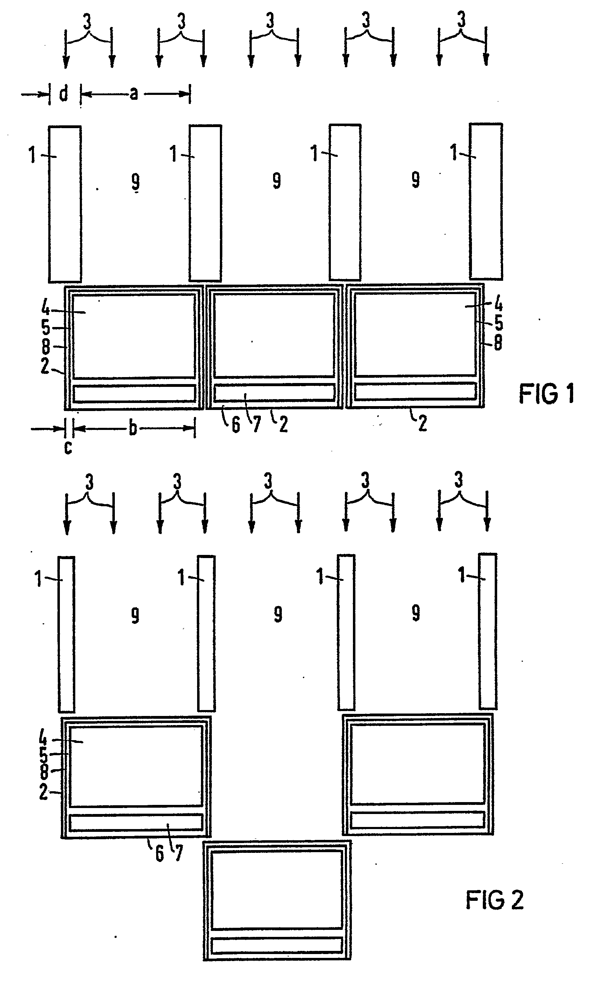

Die Figur 1 zeigt eine zur Erfindung hinführende Anordnung des Detektorsystems mit Kollimatorblechen 1 der Dicke d und linear an den Strahlungsaustrittsenden der Kollimatorschächte 9 angeordneten Detektorelementen 2. Die Detektorelemente 2 sind in diesem Ausführungsbeispiel durch bekannte Szintillationsdetektoren realisiert, bei denen jedes Element 2 einen Szintillationskristall 4 enthält, dessen Breite b geringfügig größer ist als der freie Abstand a zweier benachbarter Kollimatorbleche 1. Der Szintillationskristall 4 ist seitlich und an der Seite, auf der die Strahlung auftrifft, mit einer Reflektorschicht 5 umgeben. Die dem Kollimatorblech 1 abgewandte Seite des Szintillators 4 ist durch eine in Epoxidharz 6 eingefaßte Siliziumphotodiode 7 abgedeckt. Abgeschlossen ist die Anordnung durch eine metallische Umhüllung 8 aus Aluminium. Die in dem Szintillationskristall 4 (z.B. Natrium-Jodid) absorbierten Röntgenquanten bewirken eine Emission von Lichtquanten. Diese treffen, nachdem sie den Szintillationskristall 4 und die Epoxidharzschicht 6 durchlaufen haben (direkt oder nach Reflexion an der Reflektorschicht 5), auf die Siliziumphotodiode 7 und werden dort in Form eines elektrischen Signales nachgewiesen.

Die Röntgenstrahlung 3 durchsetzt die Kollimatorschächte 9 parallel zur Richtung der Kollimatorbleche 1. Nicht parallel dazu verlaufende Röntgenstreustrahlung wird durch die stark absorbierenden Kollimatorbleche 1 weitgehend unterdrückt. Es sei c die Dicke der Kapselung, bestehend aus der Aluminiumumhüllung 8 und der Reflektorschicht 5. Wählt man 2c kleiner gleich d, so können die seitlichen, für den Nachweis der Röntgenstrahlung nicht wirksamen Gebiete der Aluminiumumhüllung 8 und der Reflektorschicht 5 in den Strahlungsschatten der Kollimatorbleche 1 gelegt werden. Ist die aktive Detektorbreite b größer als der Raum a zwischen den Kollimatorblechen 1, so hat das zusätzlich den Vorteil, daß die bei diesem Detektor üblichen, weniger empfindlichen seitlichen Randschichten des Szintillators 4 ebenfalls in die Strahlungsschatten der Kollimatorbleche 1 fallen.

Da sich die einzelnen Detektorelemente 2 aufgrund einer zu geringen Dicke der Kollimatorbleche in manchen Fällen nicht mehr linear nebeneinander anordnen lassen, ohne daß die Umhüllungen aus den Strahlungsschatten heraustreten (2c größer d), sind die Elemente 2 nach der Erfindung alternierend auf zwei Linien gemäß Figur 2 positioniert. Ein Vorteil dieser Anordnung ist, daß bei Hintereinanderstellung der seitlichen Detektorkapselungen gemäß dieser Anordnung die Dicke der Kollimatorbleche weiter reduziert werden kann, andererseits die Breite b der Szintillatoren und der Siliziumphotodioden größer gewählt werden kann. Die mechanische Stabilität dieser Teile ist also besser als in der Anordnung nach Figur 1.

## Patentansprüche

Röntgendetektorsystem mit einer Reihe von Detektorelementen (2), denen die Kollimatorschächte (9) eines Kollimators derart zugeordnet sind, daß die von einer Röntgenstrahlenquelle ausgesandte Röntgenstrahlung (3) die Kollimatorschächte (9) durchdringt und der strahlungselektrische Wandler (4, 7) jedes Detektorelementes (2), der etwa so breit ist wie ein Kollimatorschacht (9), die gesamte einen Kollimatorschacht (9) durchsetzende Strahlung auffängt, wobei die Detektorelemente (2) in Strahlungsrichtung hinter den Kollimatorschächten (9) angeordnet sind und der Wandler (4, 7) jedes Detektorelementes (2) aus einem Szintillator (4) und einem ihm nachgeschalteten Element besteht, dadurch gekennzeichnet, daß das dem Szintillator (4) nachgeschaltete Element eine Siliziumphotodiode (7) ist, daß die Kollimatorschächte (9) von Kollimatorblechen (1) gebildet sind, daß jeder Szintillator (4) zusammen mit der zugeordneten Siliziumphotodiode (7) ein eigenes, gemeinsames Gehäuse (8) besitzt, daß die in Richtung der Kollimatorschächte (9) ausgerichteten seitlichen Gehäusewandungen im Strahlungsschatten der Kollimatorbleche (1) liegen und daß ein erstes von zwei quer zur Strahlungsrichtung nebeneinander angeordneten Detektorelementen (2) in Strahlungsrichtung um mindestens eine Seitenwandlänge nach hinten versetzt neben dem zweiten Detektorelement (2) angeordnet ist, derart, daß die aufeinanderfolgenden seitlichen Gehäusewandungen der zwei Detektorelemente (2) im Strahlungsschatten des zugeordneten Kollimatorbleches (1) hintereinander angeordnet sind.

## Claims

X-ray detector system having a series of detector elements (2) with which are associated the collimator channels (9) of a collimator so that the X-radiation (3) emitted from an X-ray source penetrates the collimator channels (9), and the radiation-electrical transducer (4, 7) of each detector element (2), which is approximately as wide as a collimator channel (9), picks up the entire radiation penetrating a collimator channel (9), the detector elements (2) being arranged behind the collimator channels (9), in the radiation direction, and the transducer (4, 7) of each detector element (2) comprising a scintillator (4) and an element connected to it on the outlet side, characterised in that the element connected to the scintillator (4) on the outlet side is a silicon photodiode (7), in that the collimator channels (9) are formed from collimator plates (1), in that each scintillator (4) together with the associated silicon photodiode (7) has its own common housing (8), in that the lateral housing walls aligned in the direction of the collimator channels (9) lie in radiation shadows of the collimator plates (1) and in that a first of two detector elements (2) arranged next to each other transversely to the radiation direction is arranged next to the second detector element (2) displaced rearwardly in the radiation direction by at least one side wall length, so that the consecutive lateral housing walls of the two detector elements (2) are arranged one behind the other in the radiation shadow of the associated collimator plate (1).

## Revendications

Système de détection de rayons X, avec une série d'éléments-détecteurs (2) auxquels sont associés de telle sorte des puits de collimation (9) d'un collimateur que le rayonnement X (3) émis par la source radiogène traverse les puits de collimation (9) et que transducteur rayonnement/signaux électriques (4, 7) de chaque élément-détecteur (2), qui est à peu près aussi large qu'un puits de collimation, intercepte la totalité du rayonnement qui passe par un puits de collimation (9), les éléments-détecteurs (2) étant disposés, dans le sens de propagation du rayonnement, derrière les puits de collimation (9) et le transducteur (4, 7) de chaque élément-détecteur (2) étant constitué par un scintillateur (4) et par un élément monté en aval de ce dernier, caractérisé par le fait que l'élément monté en aval du scintillateur (4) est une photodiode au silicium (7), que les puits de collimation (9) sont formés par des tôles de collimateur (1), que chaque scintillateur (4) possède, avec la photodiode au silicium (7), un boîtier commun qui lui est propre, que les parois latérales du boîtier, qui sont orientées en direction des puits de collimation (9), se situent dans la zone d'ombre du rayonnement dûe aux tôles de collimation (1), et qu'un premier de deux éléments-détecteurs (2) disposés côte-à-côte et transversalement à la direction du rayonnement, est disposé près du second élément-détecteur (2), avec un décalage vers l'arrière, dans la direction du rayonnement, sur au moins une longueur de paroi latérale, la réalisation étant telle que les parois de boîtier latérales successives des deux éléments-détecteurs (2) se situent l'un derrière l'autre dans les zones d'ombres des tôles de collimation associées (1).

FIG 1

FIG 2